# EUROPÄISCHE PATENTSCHRIFT

(11) **EP 3 303 545 B1**
(45) Veröffentlichungstag und Bekanntmachung des Hinweises auf die Patenterteilung: **07.05.2025**
(21) Anmeldenummer: 16713288.5
(22) Anmeldetag: 08.03.2016
(51) Int. Cl.: F28D 1/06, F28D 21/00, C12M 1/00, C12M 3/00, C12M 1/34, C12M 1/02

(54) **SYSTEM, VORRICHTUNG UND VERFAHREN ZUM AUFNEHMEN EINES EINWEGBEUTELS**
SYSTEM, DEVICE AND METHOD FOR RECEIVING A DISPOSABLE BAG
SYSTÈME, DISPOSITIF ET PROCÉDÉ DE RÉCEPTION D'UNE POCHE À USAGE UNIQUE

(30) Priorität: 02.06.2015 DE 102015007061
(43) Veröffentlichungstag der Anmeldung: 11.04.2018
(73) Patentinhaber: Sartorius Stedim Biotech GmbH, 37079 Göttingen (DE)
(72) Erfinder: HUSEMANN, Bernward, 37079 Göttingen (DE); TOPP-MANSKE, Simon, 34253 Lohfelden (DE); NICKEL, Bjorn, 37235 Hessisch Lichtenau (DE); LEUPOLD, Marco, 37075 Göttingen (DE)
(74) Vertreter: Novagraaf International SA
(86) Internationale Anmeldenummer: PCT/EP2016/000407
(87) Internationale Veröffentlichungsnummer: WO 2016/192824

(56) Entgegenhaltungen:
- WO-A1-2006/032084
- WO-A1-2015/039034
- DE-A1- 102005 006 055
- DE-A1- 4 028 871
- DE-U1- 7 613 603
- GB-A- 190 321 371
- US-A- 3 980 131
- US-A1- 2005 272 146
- US-A1- 2011 310 696
- US-A1- 2015 299 641

## Beschreibung

Die Erfindung betrifft ein System, eine Vorrichtung und ein Verfahren zum Aufnehmen eines Einwegbeutels.

Bioreaktoren und Pallettanks dienen als Vorrichtung zur Aufnahme und Lagerung von biologischen Medien wie z.B. Fluiden. Biologische Medien können in Einwegbeuteln bereit gestellt werden, die ein Volumen von wenigen Litern bis zu mehreren hundert Litern umfassen können. Die biologischen Medien werden innerhalb eines Einwegbeutels in den Bioreaktor eingebracht, in dem sie über einen vorbestimmten Zeitraum von üblicherweise mehreren Stunden Dauer auf einer vorbestimmbaren Temperatur temperiert werden. Weiterhin können in einem solchen Bioreaktor unterschiedliche Untersuchungen an dem biologischen Medium vorgenommen werden.

Die Handhabung eines Bioreaktors kann unter Reinraumbedingungen erfolgen, so dass besonders hohe Anforderungen an die Qualitätssicherung am Bioreaktor gestellt werden. Dabei stellen sich insbesondere hohe Qualitätsanforderungen an die Temperierung des biologischen Mediums, da sich z.B. bei Verwendung von Temperierfluiden unter Hochdruck besondere Gefahrensituationen bei der Handhabung ergeben können. Bei der Handhabung von Temperierfluiden unter Hochdruck haben wohldefinierte Sicherheitsvorkehrungen zu erfolgen.

Aus den Dokumenten US 2011/0310696 A1, WO 2015/039034 A1 und US 2005/0272146 A1 ist jeweils ein Bioreaktor bekannt, der z.B. mittels eines Fluids temperierbar ist.

Das Dokument US 2015/0299641 A1 offenbart ein Wärmetauschermodul zur Verwendung in einem chemischen, pharmazeutischen oder biologischen Reaktorsystem. Das Wärmetauschermodul ist zur Wärmeübertragung konfiguriert an das Reaktorsystem, welches einen flexiblen Einwegbehälter und mindestens eine wärmeleitende Oberfläche aufweist. Das Wärmetauschermodul weist einen Fluidzirkulationspfad auf, durch den ein Wärmeaustauschfluid zirkuliert werden kann.

Das Dokument US 3,980,131 A offenbart ein Gefäß mit einem Kulturmedium oder Laborware, welches schnell auf eine Sterilisationstemperatur gebracht wird, indem eine Mischung aus Dampf und überhitztem Wasser durch einen engen Mantel zirkuliert wird. Die Mischung aus Dampf und überhitztem Wasser wird in einem Teil des Sterilisators außerhalb des Mantels erzeugt und ohne Verwendung einer Pumpe umgewälzt. Der Sterilisationsvorgang kann mit einer automatischen Steuerung durchgeführt werden.

Der Erfindung liegt die Aufgabe zugrunde, eine Möglichkeit der eingangs genannten Art zum Aufnehmen eines Einwegbeutels mit einer verbesserten und/oder vereinfachten Temperierung bereitzustellen.

Diese Aufgabe wird durch die Gegenstände der unabhängigen Ansprüche gelöst. Bevorzugte Ausführungsbeispiele sind die Gegenstände der abhängigen Ansprüche.

Ein Aspekt betrifft ein System zum Aufnehmen eines Einwegbeutels mit einem Aufnahmebehälter mit einem Behälterinnenraum zum Aufnehmen des Einwegbeutels und einer Temperierhohlwand, die den Behälterinnenraum des Aufnahmebehälters zumindest teilweise umgibt.

Das System kann als ein Bioreaktor und/oder Pallettank ausgebildet sein, der zur Aufnahme von Einwegbeuteln mit einem Volumen von ca. 1 Liter bis ca. 2000 Litern ausgebildet sein kann, bevorzugt mit einem Volumen von ca. 200 Litern bis ca. 1000 Litern. Das System kann insbesondere zum Aufnehmen von Einwegbeuteln ausgebildet sein, in denen sich ein biologisches Medium wie ein Fluid befindet, das über einen vorbestimmten Zeitraum im System gelagert, temperiert und/oder anders untersucht werden soll. Das System weist eine Vorrichtung auf.

Der Aufnahmebehälter des Systems stellt den Behälterinnenraum bereit, der zum Aufnehmen des Einwegbeutels ausgebildet ist. Dabei kann der Behälterinnenraum zum Aufnehmen eines vorbestimmbaren Typs eines Einwegbeutels ausgebildet sein, also zum Beispiel eines Einwegbeutels eines vorbestimmbaren Herstellers und/oder mit einem vorbestimmbaren Füllvolumen. Behälterwände des Aufnahmebehälters definieren dabei den Behälterinnenraum. Dabei müssen die Behälterwände des Aufnahmebehälters den Behälterinnenraum nicht vollständig umgeben und/oder umgrenzen. So kann der Aufnahmebehälter zum Beispiel eine Rühröffnung aufweisen, durch die eine Rührvorrichtung an den Einwegbeutel im Behälterinnenraum anschließbar ist. Eine solche Rührvorrichtung ist bevorzugt am oberen Ende des Aufnahmebehälters ausgebildet. Der Aufnahmebehälter kann somit insbesondere deckellos und/oder oben offen ausgebildet sein.

Die Begriffe "oben", "unten", "seitlich", "vertikal", "horizontal", "Höhe" etc. beziehen sich im Rahmen dieser Erfindung auf das Bezugssystem der Erde, in dem das System bzw. die Vorrichtung in einer Betriebsstellung angeordnet ist.

Der Einwegbeutel wird bevorzugt so in den Aufnahmebehälter eingebracht, dass er auf einem Boden des Aufnahmebehälters aufliegt und in direktem, physikalischen Kontakt mit dem Behälterwänden des Aufnahmebehälters steht, insbesondere in Kontakt mit dem Boden des Aufnahmebehälters und/oder den an den Boden angrenzenden Behälterwänden.

Die Temperierhohlwand ist in den Behälterwänden des Aufnahmebehälters ausgebildet in einem geschlossenen Temperierkreislauf. Die Temperierhohlwand kann als Teil der Behälterwände ausgebildet sein. Die Temperierhohlwand kann als eine Doppelwand mit einer Temperierinnen- und einer Temperieraußenwand ausgebildet sein. Dabei kann die Temperierinnenwand dem Behälterinnenraum des Aufnahmebehälters zugewandt sein, und die Temperieraußenwand dem Behälterinnenraum abgewandt sein.

In einem Hohlraum zwischen der Temperierinnenwand und der Temperieraußenwand ist das Temperiermedium ausgebildet. Eine Temperiereinheit kann den Behälterinnenraum temperieren, insbesondere den Inhalt des Einwegbeutels, also z.B. das biologische Medium, mit dem Temperiermedium. Dazu kann eine Steuerung vorgesehen sein, mittels der die Temperatur und/oder der Druck des Temperiermediums in der Temperierhohlwand steuerbar und/oder einstellbar ist. Die Steuerung kann als Teil der Temperiereinheit ausgebildet sein. Die Temperiereinheit kann somit die Steuerung und/oder das in der Temperierhohlwand angeordnete Temperiermedium umfassen.

Das Innere der Temperierhohlwand kann als freier Hohlraum oder mit zumindest einem Strömungsführungselement zur Strömungsführung des Temperiermediums durch die Temperierhohlwand ausgebildet sein.

Die beiden Wände der Temperierhohlwand, also eine Temperierinnenwand und eine Temperieraußenwand, können einen Abstand von einigen Millimetern zueinander aufweisen und z.B. zwischen 5 mm und 20 mm voneinander beabstandet sein.

Die Innenseite der Temperierhohlwand, also die dem Behälterinnenraum zugewandte Temperierinnenwand, kann dabei direkt an den Behälterinnenraum angrenzend ausgebildet sein. Mit anderen Worten kann zwischen dem Behälterinnenraum und der Temperierhohlwand kein weiteres Element des Systems mehr ausgebildet sein. Mit anderen Worten kann die Temperierhohlwand den Behälterinnenraum somit zumindest teilweise unmittelbar umgeben und/oder begrenzen.

Dabei bedeutet "zumindest teilweise umgeben", dass die Temperierhohlwand den Behälterinnenraum an seiner Außenfläche zumindest zu 40% umgibt, bevorzugt zumindest zu 60% umgibt, besonders bevorzugt zumindest zu 70% umgibt, besonders bevorzugt zumindest zu 80% umgibt. Insbesondere kann dies bedeuten, dass der Behälterinnenraum vollständig bis auf einen oberen Deckelbereich und ggf. Sichtfenster und/oder eine Türöffnung von der Temperierhohlwand umgebebn und/oder umgrenzt und/oder temperiert wird.

Das Temperaturmedium ist dabei in der Temperierhohlwand auf einen Druck eingestellt, der maximal etwa 1 bar beträgt, bevorzugt maximal etwa 0,5 bar. Mit einem maximalen Druck von etwa 1 bar bzw. 0,5 bar oder weniger gilt das Temperaturmedium nicht mehr als ein Hochdruckmedium, abhängig von der anzuwendenden Richtlinie für Druckgefäße. Gemäß der ASME-Richtlinie ("American Society of Mechanical Engineers" für den amerikanischen Wirtschaftsraum) beträgt der maximale Druck 1 bar, gemäß der PED-Richtlinie ("Pressure Equipment Directive" für den europäischen Wirtschaftsraum) beträgt der maximale Druck 0,5 bar. Die Anforderungen an ein Hochdruckmedium sind besonders hoch, da bei der Handhabung und/oder Verwendung eines Hochdruckmediums besondere Sicherheitsrisiken bestehen. Durch die Verwendung eines Temperaturmediums unter einem solchen Niederdruck wird das Sicherheitsrisiko bei der Temperierung deutlich reduziert. Insbesondere kann das Temperiermedium auch unter einem Druck vorliegen, der maximal genau 0,5 bar beträgt, oder der immer kleiner als 0,5 bar ist.

Allgemein kann das Temperiermedium als ein Fluid wie zum Beispiel eine Flüssigkeit oder ein Gas ausgebildet sein, das sich in einem abgeschlossenen Temperierkreislauf befindet. Zumindest einen Teil dieses Temperierkreislaufs stellt die Temperierhohlwand des Aufnahmebehälters dar.

Gemäß einer Ausführungsform stellt die Temperiereinheit das Temperiermedium in der Temperierhohlwand in einem geschlossenen Temperierkreislauf auf einen vorbestimmten Druck von maximal etwa 1 bar oder etwa 0,5 bar ein. Die Temperiereinheit umfasst somit Einstellmittel wie eine Steuerung zum Einstellen des Drucks des Temperiermediums. Insbesondere ist die Temperiereinheit dazu ausgebildet und vorgesehen, den Druck des Temperiermediums auf maximal etwa 0,5 bar einzustellen, wodurch ein Sicherheitsrisiko reduziert wird.

Gemäß einer Ausführungsform temperiert die Temperiereinheit den Behälterinnenraum mit dem Temperiermedium, welches einen Druck von etwa 0,20 bar bis etwa 0,45 bar aufweist. In diesem Druckbereich ist das Temperaturmedium noch kein Hochdruckmedium mit den damit verbundenen Sicherheitsrisiken, weist aber trotzdem einen hinreichend hohen Druck auf, um eine ausreichende Leistung zur Temperierung des Behälterinnenraums zu gewährleisten.

Das System weist eine Vorrichtung auf zum Aufnehmen des Einwegbeutels mit dem Aufnahmebehälter, der den Behälterinnenraum zum Aufnehmen des Einwegbeutels aufweist. Die Temperierhohlwand umgibt den Behälterinnenraum des Aufnahmebehälters zumindest teilweise. Dabei ist die Temperierhohlwand dazu ausgebildet und vorgesehen ist, das Temperiermedium zum Temperieren des Behälterinnenraums unter einem Druck von maximal etwa 1 bar, bevorzugt maximal etwa 0,5 bar aufzunehmen.

Die Vorrichtung kann als ein Bioreaktor ausgebildet sein, der zur Aufnahme von Einwegbeuteln mit einem Volumen von mehreren hundert Litern ausgebildet sein kann, wie z.B. mit einem Volumen von 1 Liter bis zu 2000 Litern.

Die Vorrichtung ist als Bestandteil des Systems ausgebildet.. Damit können alle im Zusammenhang mit der Vorrichtung beschriebenen Ausführungsformen auch im System implementiert sein.

In der Vorrichtung kann das Temperiermedium in der Temperierhohlwand angeordnet sein und/oder werden. Die Temperierhohlwand ist speziell dazu ausgebildet und vorgesehen, ein Temperiermedium unter einem Maximaldruck von etwa 0,5 bar aufzunehmen. Dazu kann eine Steuerung vorgesehen sein, mittels der die Temperatur und/oder der Druck des Temperiermediums in der Temperierhohlwand steuerbar und/oder einstellbar ist. Die Steuerung kann als Teil der Vorrichtung ausgebildet sein. Die Vorrichtung kann somit die Steuerung und/oder das in der Temperierhohlwand angeordnete Temperiermedium umfassen.

Durch die Verwendung eines Temperaturmediums unter einem solchen Niederdruck wird das Sicherheitsrisiko bei der Temperierung deutlich reduziert.

Gemäß einer Ausführungsform ist die Temperierhohlwand dazu ausgebildet und vorgesehen, das Temperiermedium zum Temperieren des Behälterinnenraums unter einem Druck von etwa 0,20 bar bis etwa 0,45 bar aufzunehmen. Dabei kann das Temperiermedium in der Temperierhohlwand beim temperieren einen Druck von etwa 0,20 bar bis etwa 0,45 bar aufweisen. In diesem Druckbereich ist das Temperaturmedium noch kein Hochdruckmedium mit den damit verbundenen Sicherheitsrisiken, weist aber trotzdem einen hinreichend hohen Druck auf, um eine ausreichende Leistung zur Temperierung des Behälterinnenraums zu gewährleisten.

Gemäß einer Ausführungsform ist in der Temperierhohlwand zumindest ein Strömungsführungselement zur Strömungsführung des Temperiermediums durch die Temperierhohlwand angeordnet. Insbesondere kann hierzu eine Mehrzahl von Strömungselementen vorgesehen sein. Das Strömungselement kann als zumindest abschnittsweise im Wesentlichen geradlinig verlaufende Trennwand ausgebildet sein, die einen Strömungsweg im Inneren der Temperierhohlwand zumindest abschnittsweise definiert und/oder bestimmt. Das Strömungselement kann z.B. als eine Leitwendel durch die Temperierhohlwand ausgebildet sein. Das Strömungselement kann die Bildung eines ungleichmäßig verteilten Strömungsmusters in der Temperierhohlwand verhindern. Ohne Strömungselement könnte das Temperiermedium in der Temperierhohlwand im Wesentlichen geradlinig von einem Temperiermediumeinlass zu einem Temperiermediumauslass strömen. Durch das Strömungselement kann das Strömen durch die Temperierhohlwand umgelenkt werden. Insbesondere kann durch das oder die Strömungselement(e) die Temperierung durch das Temperiermedium verbessert werden, indem das geführte, frisch temperierte Temperiermedium im Wesentlichen die gesamte Temperierhohlwand im Wesentlichen gleichmäßig durchläuft.

Gemäß einer Ausführungsform ist das zumindest eine Strömungsführungselement so schräg ausgebildet und/oder angeordnet, dass die Strömungsführung des Temperiermediums durch die Temperierhohlwand durchgehend mit einer vertikalen Richtungskomponente erfolgt. Hierbei bedeutet schräg, dass das Strömungsführungselement nicht ausschließlich horizontal ausgebildet ist, was einen im Wesentlichen horizontalen Abschnitt der Strömungsführung bewirken würde, sondern dass das Strömungsführungselement zumindest auch eine vertikale Ausbreitung aufweist. Dies bewirkt eine Strömungsführung durch die Temperierhohlwand mit zumindest einer vertikalen Komponente. Bevorzugt erfolgt die Strömungsführung mit sowohl einer vertikalen als auch einer horizontalen Richtungskomponente, um das Temperiermedium möglichst gleichmäßig und effizient durch die Temperierhohlwand zu führen. Hierbei kann die horizontale Komponente der Strömungsführung größer als die vertikale Komponente ausgebildet sein, bevorzugt sogar zumindest doppelt oder zumindest dreimal so groß. Insbesondere kann die Strömungsführung von einem an einem unteren Ende der Temperierhohlwand angeordneten Temperiermediumeinlass zu einem am oberen Ende der Temperierhohlwand angeordneten Temperiermediumauslass geführt sein und/oder umgekehrt. Um eine Bildung von Luftbläschen in der Strömungsführung zu vermeiden, weist die Strömungsführung im Wesentlichen durchgehend eine vertikale Richtungskomponente auf. Hierbei kann eine Störung der Temperierung durch Lufteinschlüsse reduziert werden, insbesondere unabhängig von einer Strömung und/oder einer Strömungsrichtung des Temperiermediums. Dadurch kann die Temperierwirkung verbessert werden.

Gemäß einer Ausführungsform ist an einem oberen Ende der Temperierhohlwand eine Sammelkammer mit einem Entlüftungsausgang und einem davon separaten Temperiermediumauslass angeordnet. Der separate Temperiermediumauslass kann z.B. als ein Überlauf und/oder Rücklauf ausgebildet sein, über den das Temperiermedium aus der Temperierhohlwand herausgeführt wird. Der Entlüftungsausgang kann an einem oberen Ende der Sammelkammer angeordnet sein und somit an einer Position ansetzen, an der sich Luft in der Temperierhohlwand sammelt. Dadurch wird eventuell im Kreislauf vorhandene Luft nicht immer wieder durch den ganzen Kühlkreislauf geleitet, sondern kann möglichst einfach entlüftet werden. Der Entlüftungsausgang kann auf den in der Temperierhohlwand eingestellten Druck abgestimmt sein. Über den Entlüftungsausgang kann auch ein Solldruck in der Temperierhohlwand gesteuert und/oder geregelt werden.

Gemäß einer Ausführungsform ist die Temperierhohlwand so ausgebildet, dass sie zumindest ein unteres Drittel des Behälterinnenraums im Wesentlichen vollständig umgibt und/oder begrenzt. Im unteren Drittel des Behälterinnenraums befindet sich der Boden des Aufnahmebehälters sowie die an den Boden angrenzenden Behälterwände. Zumindest an diesem unteren Drittel ist die Temperierhohlwand in den Behälterwänden des Aufnahmebehälters ausgebildet. An dem unteren Drittel kann eine stabile Behälterwanne ausgebildet sein. Somit wird zumindest das untere Drittel des Behälterinnenraums vollumfänglich, d.h. entlang des gesamten Seitenwandumfangs des Aufnahmebehälters, und im Wesentlichen vollständig bis auf ggf. in der Behälterwand angeordnete Bodensichtfenster von dem Temperiermedium temperiert.

Gemäß einer Ausführungsform ist die Temperierhohlwand so ausgebildet, dass sie den Behälterinnenraum bis über eine vorbestimmte Füllstandhöhe im Aufnahmebehälter hinaus temperiert und/oder umgibt und/oder begrenzt. Die Vorrichtung und insbesondere der Aufnahmebehälter sind zur Aufnahme eines vorbestimmbaren Einwegbeutels ausgebildet. Dieser Einwegbeutel umfasst ein vorbestimmbares Volumen eines biologischen Mediums. Damit ergibt sich die vorbestimmte Füllstandhöhe im Aufnahmebehälter entsprechend der Höhe des Füllstands des biologischen Mediums im Aufnahmebehälter, wenn einer der vorbestimmbaren Einwegbeutel mit biologischem Medium im Behälterinnenraum angeordnet ist.

Die Temperierhohlwand umgibt und/oder begrenzt den Behälterinnenraum somit nicht nur bis zu dieser vorbestimmten Füllstandhöhe, sondern nach oben über diese Füllstandhöhe hinaus. Üblicherweise wird der obere Teil wie z.B. ein Deckel eines Bioreaktors nicht mehr temperiert, weil das biologische Medium nicht bis an den Deckel des Bioreaktors aufgefüllt ist und somit nicht am Deckel anliegt. Somit würde bei Temperierung des Deckels bzw. des oberen Teils des Bioreaktors nicht das biologische Medium, sondern lediglich in der Vorrichtung befindliche Luft temperiert werden, die ggf. durch eine Öffnung im Behälter entweichen kann. Deswegen wird herkömmlicherweise eine Temperierung von im Bioreaktor befindlicher Luft vermieden und nur bis maximal zur Füllstandhöhe temperiert. Insbesondere beim Rühren des biologischen Mediums im Bioreaktor kann das biologische Medium jedoch über die vorbestimmte Füllstandhöhe hinaus im Aufnahmebehälter ansteigen. Dies erfolgt insbesondere bei einer Trichterbildung aufgrund von Zentrifugalkräften bei einer Rührbewegung. Die Temperierhohlwand ist bei dieser Ausführungsform der Vorrichtung deutlich über die vorbestimmte Füllstandhöhe hinaus ausgebildet und ist somit auch in der Lage, das biologische Medium im Inneren des Einwegbeutels selbst während einer Rührbewegung bis über die vorbestimmte Füllstandhöhe hinaus zu temperieren. Dadurch wird die Temperierung verbessert.

In einer Weiterbildung dieser Ausführungsform ist die Temperierhohlwand so ausgebildet, dass sie den Behälterinnenraum mindestens etwa 1 cm und maximal etwa 20 cm, bevorzugt von etwa 5 cm bis etwa 12 cm über die vorbestimmte Füllstandhöhe im Aufnahmebehälter hinaus temperiert und/oder umgibt und/oder begrenzt. Diese Höhe hat sich als geeigneter Mittelwert erwiesen sowohl im Hinblick auf ein möglichst effizientes Temperieren des biologischen Mediums, als auch im Hinblick auf ein Minimieren der Leistungsverluste durch Temperieren von Luft im oberen Bereich des Aufnahmebehälters.

Gemäß einer Ausführungsform weist die Vorrichtung zumindest ein Entlastungsventil zum Einstellen und/oder Begrenzen des Drucks des Temperiermediums in der Temperierhohlwand auf. Das Entlastungsventil kann als Bestanteil einer Steuerung und/oder einer Temperiereinheit ausgebildet sein. Das zumindest eine Entlastungsventil bewirkt, dass das Temperiermedium in der Temperierhohlwand einen vorbestimmbaren Solldruck nicht übersteigt, z.B. den Solldruck von etwa oder genau 0,5 bar.

Gemäß einer Ausführungsform weist die Vorrichtung eine elektrische Heizung im Wärmeaustausch mit dem Temperiermedium auf. Die elektrische Heizung kann über eine Steuerung des Temperiermediums angesteuert und/oder eingestellt werden. Die elektrische Heizung kann als eine interne Heizung ausgebildet sein, die z.B. unmittelbar angrenzend an die Temperierhohlwand, im Inneren der Temperierhohlwand, und/oder im Temperierkreislauf des Temperiermediums ausgebildet sein kann. Die elektrische Heizung kann zum Beispiel als eine Heizpatrone ausgebildet sein. Die elektrische Heizung dient zum Einstellen der Temperatur des Temperiermediums und somit zum Einstellen einer Zieltemperatur für den Behälterinnenraum.

Gemäß einer Ausführungsform ist das Temperiermedium durch einen externen Wärmetauscher leitbar. Der Wärmetauscher kann als separates Bauteil oder als ein Bestandteil der Vorrichtung ausgebildet sein. Der Wärmetauscher kann dazu ausgebildet und vorgesehen sein, das Temperiermedium sowohl zu kühlen als auch zu erhitzen. Im Wärmetauscher kann ein Wärmeaustausch mit einem externen Temperiermedium vorgesehen sein und/oder erfolgen, das z.B. unter Hochdruck vorliegen kann. Durch Nutzung des Wärmetauschers können auch externe Temperiermedien verwendet werden, die z.B. für eine Durchleitung durch den Aufnahmebehälter zu aggressiv wären. Besondere Sicherheitsvorkehrungen haben hierbei lediglich zum Überwachen und/oder Kontrollieren des externen Temperiermediums zu erfolgen, nicht jedoch zur Überwachung des internen Temperaturmediums in der Temperierhohlwand. Dies vereinfacht die Handhabung des internen Temperiermediums. Das externe Temperiermedium kann unter hohen Drücken von bis zu 10 bar vorliegen, um einen hohen Wirkungsgrad beim Wärmeaustausch zu bewirken. In dieser Ausführungsform können eine oder mehrere isolierte Temperierleitungen vorgesehen sein, in denen das interne Temperiermedium zu dem externen Wärmetauscher hin leitbar ist, und von dort wieder zurück zur Temperierhohlwand.

Gemäß einer Ausführungsform ist an der dem Behälterinnenraum abgewandten Seite der Temperierhohlwand, also z.B. an der Temperieraußenwand, eine Isolierung zumindest teilweise ausgebildet, die die Temperierhohlwand nach außen isoliert. Bevorzugt ist die Isolierung so ausgebildet, dass sie die Temperierhohlwand vollständig nach außen isoliert. Dies reduziert einen Energieverlust durch Wärmeübertrag des Temperiermediums an die Außenluft, also Luft außerhalb des Behälterinnenraums. Gleichzeitig wird sichergestellt, dass die Temperatur des Temperiermediums im Wesentlichen ausschließlich nach innen, also in den Behälterinnenraum und somit an das im Einwegbeutel befindliche biologische Medium abgegeben wird. Die Isolierung kann als Luftschicht, als Vakuum, oder als ein Dämmmaterial wie z.B. Dämmwolle ausgebildet sein. Die Isolierung kann so ausgebildet sein, dass zumindest die Temperierhohlwand vollumfänglich nach außen isoliert ist.

In einer Weiterbildung dieser Ausführungsform ist die Isolierung zumindest teilweise in einer Isolierhohlwand angeordnet, die an der dem Behälterinnenraum abgewandten Seite der Temperierhohlwand ausgebildet ist. Die Behälterwand des Aufnahmebehälters weist somit zumindest teilweise einen doppelten Hohlraum auf, in anderen Worten drei hintereinander folgende Trennwände in Richtung von innen nach außen. Im ersten Hohlraum vom Behälterinnenraum aus betrachtet, der Temperierhohlwand, ist das Temperiermedium angeordnet. In der zweiten Hohlwand vom Behälterinnenraum aus betrachtet, der Isolierhohlwand, ist die Isolierung angeordnet. Von innen nach außen kann somit angeordnet sein:
1. angrenzend an den Behälterinnenraum eine Temperierinnenwand,
2. daran angrenzend das Temperiermedium in der Temperierhohlwand,
3. daran angrenzend die Temperieraußenwand, die zugleich als eine Isolierinnenwand ausgebildet sein kann,
4. daran angrenzend die Isolierung in der Isolierhohlwand, und
5. daran angrenzend eine Isolieraußenwand.

Dadurch wird eine besonders effiziente Temperierung und Isolierung des Behälterinnenraums bereitgestellt.

Gemäß einer Ausführungsform weist der Aufnahmebehälter eine öffenbare Tür auf, durch die der Einwegbeutel in im Wesentlichen horizontaler Richtung in den Behälterinnenraum einbringbar ist. Die Tür weist zumindest ein Türscharnier auf, an dem die Tür drehbar gelagert ist. Die Tür ist öffenbar und gewährt im geöffneten Zustand Zugriff auf den Behälterinnenraum. Die Tür ist als ein Teil der Behälterwand des Aufnahmebehälters ausgebildet. Die Tür ist in einer Seitenwand des Aufnahmebehälters ausgebildet, und gewährt deshalb Eingriff in den Behälterinnenraum in im Wesentlichen horizontaler Richtung. Dadurch kann der Einwegbeutel auch in im Wesentlichen horizontaler Richtung in das Behälterinnere eingebracht werden, bzw. aus dem Behälterinneren herausgenommen werden. Der Rest des Aufnahmebehälters kann massiv ausgebildet sein. Insbesondere können die übrigen Seitenwände des Aufnahmebehälters formstabil ohne weitere Öffnungsmöglichkeiten ausgebildet sein. Dies erhöht die Gesamtstabilität des Aufnahmebehälters gegenüber vorbekannten, herkömmlichen Bioreaktoren, die beim Einbringen des Einwegbeutels vollständig in zwei Hälften geteilt werden. Es hat sich herausgestellt, dass bereits eine Tür ausreichend ist, um einen Einwegbeutel hinreichend komfortabel und sicher in den Aufnahmebehälter einzuführen. Vielmehr erleichtert die Tür das Einbringen des Einwegbeutels sogar. Die Tür kann als Einblatttür oder Doppeltür ausgebildet sein.

In einer Weiterbildung der Ausführungsform ist der Aufnahmebehälter im Wesentlichen in Form eines vertikal angeordneten Zylinders ausgebildet, wobei die Tür im Zylindermantel des Aufnahmebehälters ausgebildet ist über einen Zylindersektor von etwa 80° bis etwa 150°. Dabei ist die Zylinderachse des Aufnahmebehälters im Wesentlichen vertikal im Bezugssystem der Erde angeordnet. Eine Ausbildung der Tür und somit einer gleichgroßen Türöffnung über den Winkelbereich von etwa 80° bis etwa 150° ist besonders vorteilhaft in mehrerlei Hinsicht. So erstreckt sich die Türgröße über weniger als die Hälfte des Zylinderumfangs, ist also kleiner als ein Zylindersektor mit 180°. Dies gewährleistet einerseits eine hohe statische Stabilität des Aufnahmebehälters. Andererseits ermöglicht diese Türdimensionierung ein Fixieren von Zuleitungen usw. in vertikaler Ausrichtung an einander gegenüberliegenden, statischen Behälterwänden, z.B. zum Befestigen, Versorgen und/oder Steuern einer Rührvorrichtung. Andererseits ist die Tür in der genannten Türdimensionierung groß genug, um einen hinreichend komfortablen Eingriff in das Behälterinnere zu gewährleisten. Bevorzugt erstreckt sich die Tür und somit einer gleichgroßen Türöffnung über einen Zylindersektor von 90° bis 120° in der Behälterwand des Aufnahmebehälters.

In der Ausführungsform mit der Tür kann die Temperierhohlwand und/oder die Isolierung auch in der Tür ausgebildet sein. So kann z.B. die Temperierhohlwand nicht innerhalb der Tür ausgebildet sein, sondern lediglich die Isolierung. Alternativ kann die Temperierhohlwand zusätzlich zur Ausbildung in der Behälterwand des Aufnahmebehälters auch in der Tür ausgebildet sein, und zwar auf der dem Behälterinneren zugewandten Seite der Tür. Somit kann auch eine Temperierung des Behälterinnenraums an der Tür erfolgen. An der Außenseite der Tür kann zusätzlich die Isolierung ausgebildet sein, zum Beispiel wie oben beschrieben im Inneren einer Isolierhohlwand. Alternativ kann in der Tür auch lediglich eine Isolierung ausgebildet sein ohne Temperierhohlwand. Dabei kann zum Beispiel im oder am Türscharnier (oder ggf. den Türscharnieren) der Tür eine oder mehrere Temperierleitungen zum Einleiten des Temperiermediums in die Temperierhohlwand der Tür vorgesehen sein. Dadurch wird die Temperierung nicht nur an den Behälterwänden, sondern auch an der Tür selber ermöglicht, was insgesamt die Temperierwirkung erhöht und die Temperierung und/oder Isolierung des Behälterinnenraums verbessert.

Ein dritter Aspekt betrifft ein Verfahren zum Aufnehmen eines Einwegbeutels mit den Schritten:
- Aufnehmen eines Einwegbeutels in einen Behälterinnenraum eines Aufnahmebehälters, und
- Temperieren des im Behälterinnenraum aufgenommenen Einwegbehälters mittels eines in einer den Behälterinnenraum des Aufnahmebehälters zumindest teilweise umgebenden Temperierhohlwand angeordneten Temperiermediums,
- wobei das Temperiermedium in der Temperierhohlwand einen Druck von maximal etwa 1 bar, bevorzugt etwa 0,5 bar aufweist.

Das Verfahren kann zum Beispiel unter Verwendung einer Vorrichtung gemäß dem zweiten Aspekt durchgeführt werden, und/oder unter Verwendung eines Systems gemäß dem ersten Aspekt. Somit betreffen alle im Zusammenhang mit dem ersten und zweiten Aspekt gemachten Ausführungen und insbesondere die aufgeführten Ausführungsformen auch das Verfahren gemäß dem zweiten dritten.

Der Erfindung wird nachfolgend anhand von in Figuren gezeigten Ausführungsbeispielen näher beschrieben. Einzelne Merkmale der in den Figuren gezeigten Ausführungsformen können in anderen Ausführungsformen implementiert sein. Gleiche Bezugszeichen kennzeichnen dabei gleiche oder ähnliche Merkmale der Ausführungsformen. Es zeigen:
- Figur 1: in einer perspektivischen Darstellung eine Vorrichtung zum Aufnehmen eines Einwegbeutels;
- Figur 2A: in einer ersten Seitenansicht eine Vorrichtung zum Aufnehmen eines Einwegbeutels;
- Figur 2B: in einer zweiten Seitenansicht eine Vorrichtung zum Aufnehmen eines Einwegbeutels;
- Figur 3: in einer perspektivischen Darstellung eine vertikale Schnittansicht durch eine Vorrichtung zum Aufnehmen eines Einwegbeutels;
- Figur 4A: einen ersten vertikalen Querschnitt durch eine Vorrichtung zum Aufnehmen eines Einwegbeutels;
- Figur 4B: einen zweiten vertikalen Querschnitt durch eine Vorrichtung zum Aufnehmen eines Einwegbeutels;
- Figur 5A: einen ersten horizontalen Querschnitt durch eine Vorrichtung zum Aufnehmen eines Einwegbeutels;
- Figur 5B: einen zweiten horizontalen Querschnitt durch eine Vorrichtung zum Aufnehmen eines Einwegbeutel;
- Figur 6: in einer Seitenansicht einen ersten Aufnahmebehälter zum Aufnehmen eines Einwegbeutels ohne Tür und ohne Außenwand;
- Figur 7: in einer Seitenansicht einen zweiten Aufnahmebehälter zum Aufnehmen eines Einwegbeutels ohne Tür und ohne Außenwand; und
- Figur 8: in einer perspektivischen Darstellung einen Aufnahmebehälter zum Aufnehmen eines Einwegbeutels ohne Tür und ohne Außenwand.

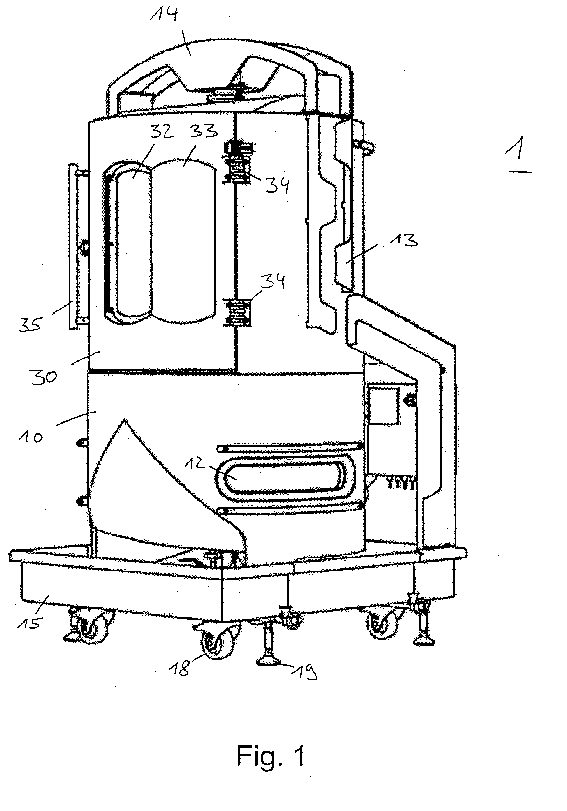

**Figur 1** zeigt in einer perspektivischen Darstellung eine Vorrichtung 1 zum Aufnehmen eines Einwegbeutels. Die in den Figuren gezeigte Vorrichtung 1 kann als ein Bestandteil eines Systems zum Aufnehmen eines Einwegbeutels ausgebildet sein.

Die Vorrichtung 1 weist einen Aufnahmebehälter 10 auf, der im Wesentlichen die Form eines vertikal angeordneten Zylinders aufweist, d.h. dessen Zylinderachse im Wesentlichen vertikal angeordnet ist. Der Aufnahmebehälter 10 weist einen Behälterinnenraum auf, in den ein Einwegbeutel eingebracht werden kann, der zum Beispiel ein biologisches Medium beinhalten kann. Das biologische Medium im Einwegbeutel wird im Behälterinnenraum des Aufnahmebehälters 10 über einen vorbestimmbaren Zeitraum gelagert. Während sich der Einwegbeutel mit dem biologischen Medium im Inneren des Aufnahmebehälters 10 befindet, können sich unterschiedliche Reaktionen mit oder an dem biologischen Medium ereignen. Somit kann die Vorrichtung 1 auch als Bioreaktor ausgebildet sein.

Zur Beobachtung des biologischen Mediums sind in den Seitenwänden ein oder mehrere Sichtfenster ausgebildet, durch das bzw. die von außen durch die Behälterwand hindurch in den Behälterinnenraum des Aufnahmebehälters 10 hineingeblickt werden kann, um das biologische Medium zu beobachten. Die Vorrichtung 1 weist dazu im unteren Drittel zwei Bodensichtfenster 12 auf, sowie ein Türsichtfenster 32. Die Bodensichtfenster 12 sind im Wesentlichen in Form eines länglichen Ovals ausgebildet, dessen lange Ovalachse im Wesentlichen horizontal entlang der gekrümmten Zylinderaußenwand des Aufnahmebehälters 10 ausgerichtet ist. Das Türsichtfenster 32 ist im Wesentlichen in Form eines länglichen Rechtecks ausgebildet, wobei deren längere Seiten im Wesentlichen vertikal ausgerichtet sind und in der Mitte einer Einblatttür 30 in der Behälterwand des Aufnahmebehälters 10 ausgebildet sind.

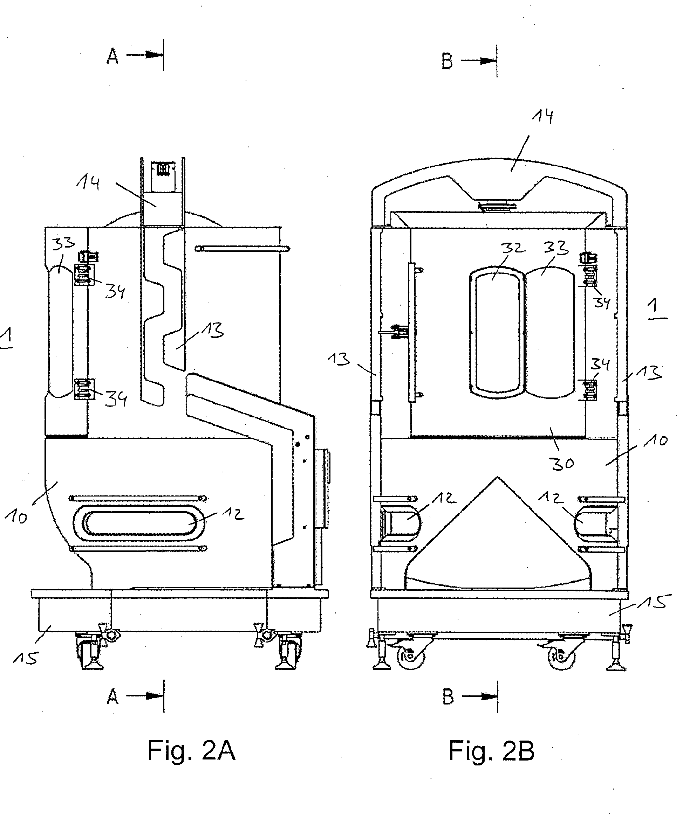

**Figuren 2A und 2B** zeigen zusammen mit Figur 1 unterschiedliche Ansichten der Vorrichtung 1. So zeigt z.B. Figur 2B eine frontale Seitenansicht auf die Einblatttür 30. Die Einblatttür 30 erstreckt sich in der Breite, also in horizontaler Richtung, in etwa über ein Zylindersegment des Aufnahmebehälters 10 von ca. 100°. In horizontaler Richtung erstreckt sich die Einblatttür 30 von zwei Türscharnieren 34 entlang des Zylindermantels bis zu einem Türgriff 35 am gegenüberliegenden Türende. Die Einblatttür 30 ist im Wesentlichen in den oberen zwei Dritteln des Aufnahmebehälters 10 ausgebildet, während das untere Drittel des Aufnahmebehälters 10 im Wesentlichen in Form einer steifen Bodenschale ausgebildet ist, die selber nicht öffenbar ausgebildet ist. Die Einblatttür 30 ist um die Türscharniere 34 drehbar und somit öffenbar. Ist die Einblatttür 30 geöffnet, so ist in dem Aufnahmebehälter 10 an einer seitlichen Position eine Türöffnung ausgebildet, durch die ein Zugriff auf den Behälterinnenraum des Aufnahmebehälters 10 ermöglicht wird. Durch die Türöffnung kann zum Beispiel der Einwegbeutel von einer seitlichen Richtung her, also im Wesentlichen in einer horizontalen Bewegungsrichtung, in den Behälterinnenraum des Aufnahmebehälters 10 eingeführt werden.

Die Vorrichtung 1 ist auf Rollen 18 rollbar gelagert, auf denen die Vorrichtung durch einen Raum geschoben werden kann. Zusätzlich zu den Rollen 18 kann die Vorrichtung 1 am unteren Ende Fixierfüße 19 aufweisen, die zum Fixieren und korrektem Ausrichten der Vorrichtung 1 auf unebenen Böden dienen.

Der Aufnahmebehälter 10 ist nach oben offen ausgebildet. An Stelle eines Zylinderdeckels weist der Aufnahmebehälter 10 eine Rühröffnung auf. Oberhalb des nach oben offenen Aufnahmebehälters 10 ist eine Rührvorrichtung 14 ausgebildet, über die ein Rührstab durch die Rühröffnung hindurch mit dem Einwegbeutel so verbunden werden kann, dass das Innere des Einwegbeutels durchmischt werden kann. Der Rührstab kann im Inneren des Einwegbeutels angeordnet sein und über eine Kopplung bzw. Kupplung mit der Rührvorrichtung 14 verbunden werden. Die Rührvorrichtung 14 ist zentral über dem Aufnahmebehälter 10 ausgebildet und wird von einer Trägerbrücke getragen, die auf einem oberen Rand des Aufnahmebehälters 10 an einander gegenüberliegenden Seitenwänden des Aufnahmebehälters 10 aufliegt.

Figur 2A zeigt eine Seitenansicht direkt auf eine der beiden Kabelführungen 13. Die zweite der beiden Kabelführungen 13 ist an der gegenüberliegenden Außenwand des Aufnahmebehälters 10 angeordnet. Die Seitenansicht der Figur 2A zeigt die Vorrichtung 1 in einer Position, die gegenüber der Seitenansicht der Figur 2B um 90° gedreht ist.

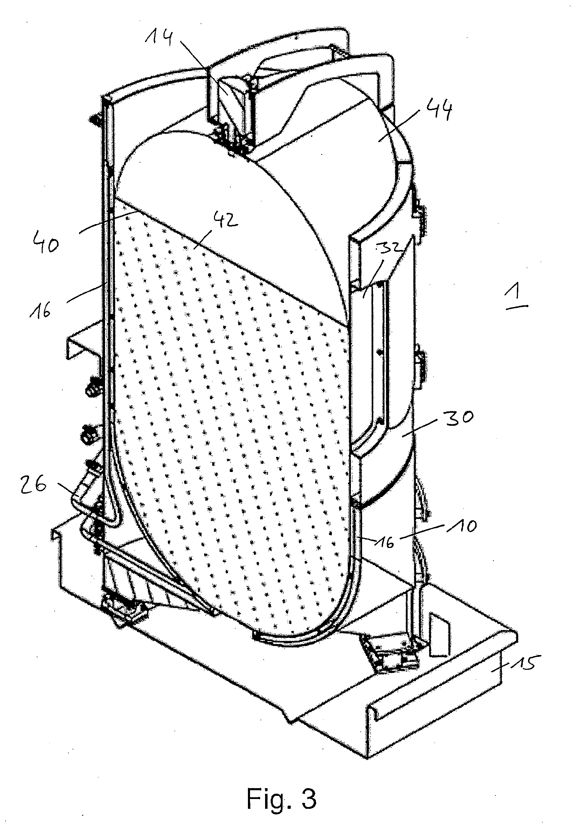

**Figur 3** zeigt in einer perspektivischen Darstellung eine Ansicht eines vertikalen Schnitts durch die Vorrichtung 1. In Figur 3 gezeigt z.B. ist ein Einwegbeutel 44, genauer ein Schnitt durch diesen Einwegbeutel 44, der im Behälterinnenraum des Aufnahmebehälters 10 angeordnet ist. Im Behälterinnenraum des Aufnahmebehälters 10 und zugleich auch im Inneren des Einwegbeutels 44 ist ein biologisches Medium 42 angeordnet, das bis auf Höhe einer vorbestimmten Füllstandhöhe 40 gefüllt ist. Das biologische Medium 42 erstreckt sich vom Boden des Aufnahmebehälters 10 bis hin zur Füllstandhöhe 40 und füllt somit das gesamte Innenvolumen des Aufnahmebehälters 10 bis hin zur Füllstandhöhe 40, abzüglich des Volumens der Wände des Einwegbeutels 44, die jedoch sehr dünn ausgebildet sind und kaum ins Gewicht fallen.

Der Einwegbeutel 44 wird durch eine Behälterwand 16 des Aufnahmebehälters 10 in Form gehalten, die sich vom abgerundeten Boden des Aufnahmebehälters 10 bis über die Füllstandhöhe 40 hinaus nach oben erstrecken. Zumindest entlang der oberen Hälfte, bevorzugt entlang der oberen zwei Drittel des Aufnahmebehälters 10 kann sich die Behälterwand 16 im Wesentlichen in vertikaler Richtung senkrecht nach oben erstrecken.

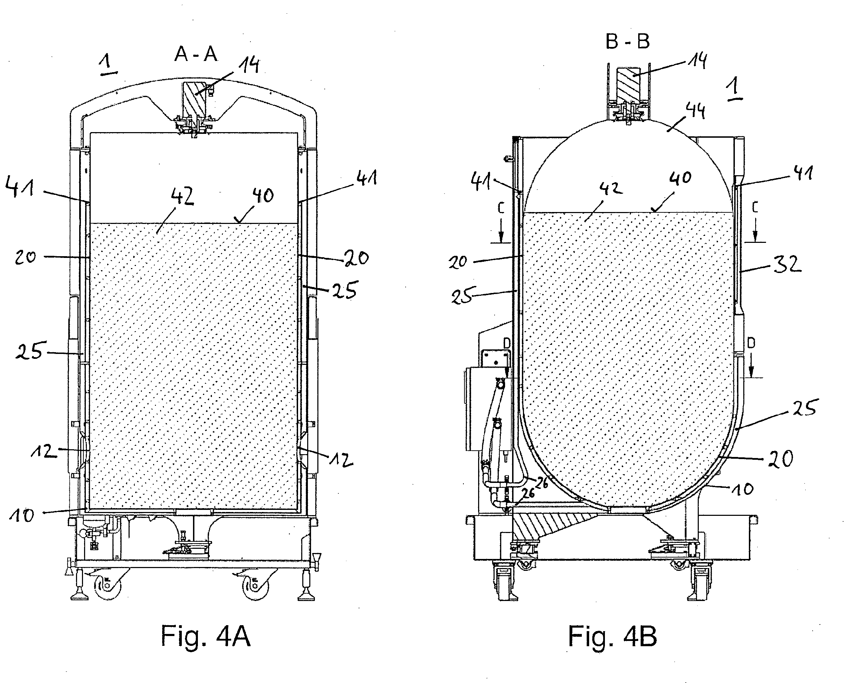

**Figuren 4A und 4B** zeigen jeweils einen Querschnitt vertikal durch die Vorrichtung 1 hindurch. Eine Schnittebene A-A, die in Figur 4A gezeigt ist, ist in Figur 2A gekennzeichnet und erstreckt sich in vertikaler Richtung durch den oberen Teil der Kabelführung 13 hindurch sowie durch die Zylindermitte des Aufnahmebehälters 10. Der in Figur 4B gezeigte Querschnitt erfolgt in einer Schnittebene B-B, die in Figur 2B gekennzeichnet ist. Die Schnittebene B-B ist senkrecht zur Schnittebene A-A angeordnet, in einer vertikalen Richtung durch die Zylinderachse des Aufnahmebehälters 10 sowie durch die Mitte der Einblatttür 30 hindurch.

In den Figuren 4A und 4B ist das Innere der Behälterwände 16 näher gezeigt. An den Innenwänden des Aufnahmebehälters 10 ist eine Temperierhohlwand 20 angeordnet, in der ein (in den Figuren nicht gezeigtes) Temperiermedium angeordnet ist. Das Temperiermedium wird auf einen Niedrigdruck kleiner als 0,5 bar geregelt. Die Temperierhohlwand 20 erstreckt sich über den gesamten Boden des Aufnahmebehälters 10 und entlang der Behälterwände vom Behälterboden nach oben bis über die Füllstandhöhe 40 hinaus bis zu einer Temperierhöhe 41. Die Temperierhöhe 41 ist im Wesentlichen 1 cm bis 20 cm vertikal oberhalb der Füllstandhöhe 40 angeordnet.

Das biologische Medium 42 steht in direktem Wärmekontakt zu der Temperierhohlwand 20, von der es lediglich über die dünne Beutelwand des Einwegbeutels 44 getrennt ist. Über das Temperiermedium kann das biologische Medium auf eine vorbestimmbare Temperatur geregelt werden.

Die Vorrichtung 1 kann insbesondere dazu ausgebildet und vorgesehen sein, den Behälterinnenraum auf eine vorbestimmbare Solltemperatur von etwa 0°C bis etwa 80°C, bevorzugt von etwa 20°C bis etwa 40°C zu temperieren.

Die Temperierhohlwand 20 umgibt den Behälterinnenraum des Aufnahmebehälters 10 bis über die Füllstandhöhe 40 hinaus beinahe vollständig. "Beinahe vollständig umgeben" bedeutet in dem in den Figuren gezeigten Ausführungsbeispiel, dass die Temperierhohlwand 20 den Behälterinnenraum bis zur Temperierhöhe 41 hin vollständig bis auf diejenigen Positionen umgibt, an denen die Bodensichtfenster 12 angeordnet sind und an der die Einblatttür 30 angeordnet ist. An den Positionen der Bodensichtfenster kann eine Glasscheibe angeordnet sein, die Sicht auf den Behälterinnenraum und insbesondere das im Aufnahmebehälter 10 befindliche biologische Medium gewährt (und gegebenenfalls eine entsprechende Verdunklung ohne Temperierung). An der Position der Einblatttür 30 befindet sich eine Aussparung in Form der Türöffnung in der Temperierhohlwand 20. In einer alternativen Ausführungsform kann auch an der Innenseite der Einblatttür 30 eine Temperierhohlwand ausgebildet sein, die über an den Türscharnieren 34 angeordnete Temperierleitungen mit dem Temperiermedium versorgt wird.

Allgemein kann "beinahe vollständig umgeben" bedeuten, dass die Temperierhohlwand 20 den Behälterinnenraum bis zur Temperierhöhe 41 hin vollständig bis auf einige wenige vorbestimmte Positionen umgibt. Diese einigen wenigen Positionen können die Positionen sein, an denen Sichtfenster in der Behälterwand des Aufnahmebehälters 10 angeordnet sind, und gegebenenfalls an der die Einblatttür 30 angeordnet ist. Im Allgemeinen begrenzt die Temperierhohlwand 20 den Behälterinnenraum nicht an dessen oberen Ende.

Die Temperierhohlwand 20 ist an ihrer Außenseite von einer Isolierhohlwand 25 umgeben, in der sich eine Isolierung befindet. Die Isolierhohlwand 25 umgibt den Behälterinnenraum des Aufnahmebehälters 10 beinahe vollständig vom Boden des Aufnahmebehälters 10 bis hin zum oberen Ende der Behälterwand 16 (vgl. dazu auch Figur 3). Die Isolierhohlwand 25 isoliert sowohl den Behälterinnenraum, als auch insbesondere die Temperierhohlwand 20 nach außen hin. Durch die in der Isolierhohlwand 25 angeordnete Isolierung wird eine gerichtete Temperierung mittels des Temperiermediums nach innen hin zum Behälterinnenraum bereitgestellt, was die Energieeffizienz der Vorrichtung 1 erhöht.

In Figur 4B sind Temperierleitungen 26 gezeigt, die mit dem Inneren der Temperierhohlwand 20 verbunden sind. Anschlüsse bzw. Isolierleitungen zum bereitstellen eines Vakuums in der Isolierhohlwand sind in den Figuren nicht gezeigt.

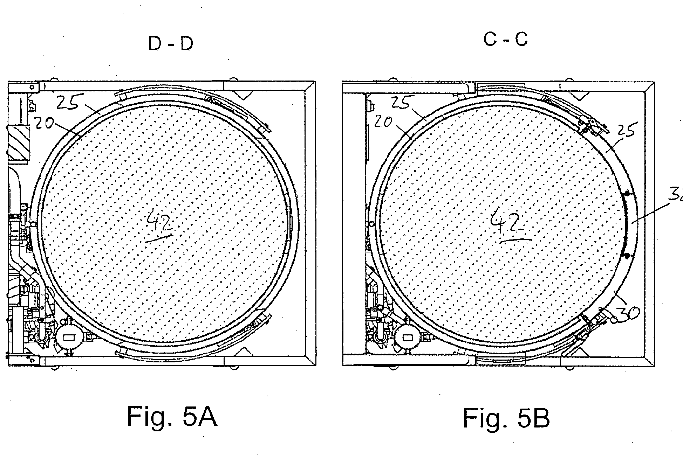

**Figuren 5A und 5B** zeigen jeweils einen Querschnitt durch die Vorrichtung 1 in einer horizontalen Schnittrichtung, und zwar durch unterschiedliche hohe Schnittebenen D-D und C-C, die in Figur 4B gekennzeichnet sind.

Figur 5A zeigt einen horizontalen Querschnitt durch das untere Drittel des Aufnahmebehälters 10, in dem die Temperierhohlwand 20 den Behälterinnenraum und somit das darin angeordnete biologische Medium 42 vollumfänglich (bis auf die Bodensichtfenster) von außen umgibt und temperiert. Figur 5A zeigt somit einen horizontalen Querschnitt durch die untere, nicht öffenbare Bodenwanne des Aufnahmebehälters 10. Die Temperierhohlwand 20 ist dabei in dem in Figur 5A gezeigten Querschnitt vollständig von der Isolierhohlwand 25 umgeben, und somit von der darin befindlichen Isolierung isoliert.

Der in Figur 5B gezeigte horizontale Querschnitt C-C ist oberhalb des in Figur 5A gezeigten Querschnitts D-D angeordnet. In der Höhe des Querschnitts C-C ist bereits die Einblatttür 30 ausgebildet. An der Position der Einblatttür 30, also an der Position der Türöffnung in der Behälterwand 16, ist die Temperierhohlwand 20 unterbrochen. An der Einblatttür 30 selber erfolgt somit keine Temperierung des biologischen Mediums 42. An der Stelle der Einblatttür 30 ist jedoch eine Isolierung in der Isolierhohlwand 25 ausgebildet, die das biologische Medium 42, also den Behälterinnenraum des Aufnahmebehälters 10, nach außen isoliert. Die Isolierhohlwand 25 ist lediglich an der Position des Türsichtfensters 32 unterbrochen, an der der Behälterinnenraum lediglich von einer Glasscheibe begrenzt ist.

Die Isolierung isoliert den Aufnahmebehälter 10 somit beinahe vollständig, insbesondere vom Behälterboden bis über der Füllstandhöhe hinaus vollständig bis auf die im Aufnahmebehälter angeordneten Sichtfenster, im Ausführungsbeispiel die Bodensichtfenster 12 und das Türsichtfenster 32. Die Isolierung kann insbesondere bis zur Temperierhöhe 41, bevorzugt bis zur Oberkante der Behälterwände 16 ausgebildet sein.

Die Temperierhöhe 41 übersteigt sowohl die vorbestimmte Füllstandshöhe 40, als auch die Kontaktfläche des Einwegbeutels 44 mit den Behälterwänden 16 des Aufnahmebehälters 10.

Der Einwegbeutel 44 wird nach der Verwendung zum Beispiel über ein unterhalb der Vorrichtung 1 angeordneten Auslass entleert und kann anschließend vollständig entsorgt werden. Durch Verwendung des Einwegbeutels 44 kann eine Reinigung der Vorrichtung 1 eingespart werden, bzw. wesentlich schneller erfolgen.

Der Auffangbehälter 15 dient als Auffangelement, falls aus der Vorrichtung 1 z.B. durch ein Leck im Einwegbeutel biologisches Medium austreten sollte.

Als Isolierung in der Isolierhohlwand kann Luft, ein Vakuum, eine Dämmwolle, eine Glaswolle, eine Steinwolle, oder ein ähnliches Isoliermaterial verwendet werden.

Die Vorrichtung 1 weist möglichst wenig Wärmebrücken auf, also durchgehende Metallverbindung vom Behälterinnenraum zum Außenraum, welche die Temperierung des Behälterinnenraums verlangsamen würden. Die Vorrichtung 1 weist lediglich statisch zwingend erforderliche Wärmebrücken auf.

Das Temperiermedium befindet sich im Inneren eines abgeschlossenen Temperiersystems, das das Innere der Temperierhohlwand 20 umfasst. Die Regelung und/oder Steuerung der Temperatur des Temperiermediums kann über eine interne, elektrisch betriebene Heizvorrichtung erfolgen sowie optional oder alternativ über einen externen Wärmetauscher. Über den externen Wärmetauscher kann sowohl eine Kühlung als auch eine Erwärmung des Temperiermediums unabhängig von der internen elektrischen Heizvorrichtung des Temperiersystems erfolgen.

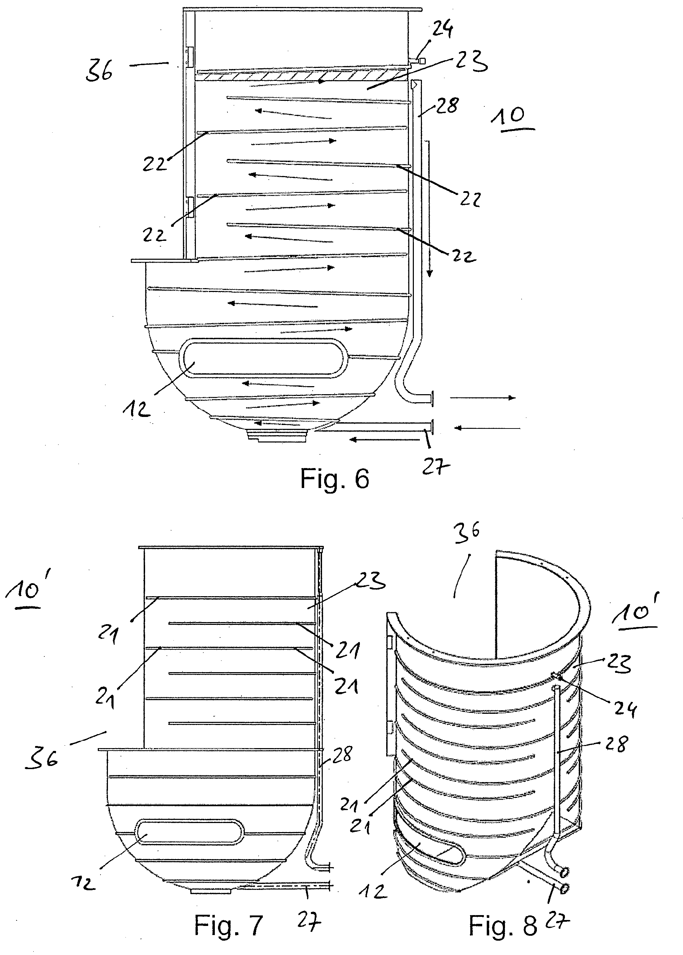

**Figur 6** zeigt in einer Seitenansicht ein erstes Ausführungsbeispiel des Aufnahmebehälters 10 zum Aufnehmen eines Einwegbeutels ohne Einblatttür 30 und ohne Außenwand. Mit anderen Worten zeigt Figur 6 einen Einblick ins Innere der Temperierhohlwand 20. Gezeigt ist somit nur eine Innenwand des Aufnahmebehälters 10 ohne seine Außenwand, die die Temperierhohlwand 20 nach außen hin begrenzt und abschließt. Ebenfalls nicht gezeigt ist die Isolierhohlwand 25, die die Temperierhohlwand 20 von außen umgrenzen kann.

Im Inneren der Temperierhohlwand 20 weist der Aufnahmebehälter 10 mehrere Strömungsführungselemente 22 auf, die in der gezeigten Seitenansicht in eine im Wesentlichen horizontale Richtung durch die Temperierhohlwand 20 hindurch verlaufend angeordnet sind. Die Strömungsführungselemente 22 sind so ausgebildet, dass sie sowohl an der Innenwand als auch an der Außenwand der Temperierhohlwand 20 dicht angrenzen. Die Strömungsführungselemente 22 können z.B. sowohl mit der Innenwand als auch mit der Außenwand der Temperierhohlwand 20 verschweißt sein.

Dadurch definieren die Strömungsführungselemente 22 im Inneren der Temperierhohlwand 20 einen Strömungskanal und/oder eine Strömungsführung für das Temperiermedium, was in Fig. 6 durch Pfeile gekennzeichnet ist.

An einem unteren Ende weist die Temperierhohlwand 20 einen Temperiermediumeinlass 27 auf, der in Form eines Anschlussrohres ausgebildet sein kann. Über den Temperiermediumeinlass 27 kann das Temperiermedium ins Innere der Temperierhohlwand 20 eingeführt und/oder eingelassen werden. Das Temperiermedium wird von dem unteren Ende der Temperierhohlwand 20 an den Strömungsführungselementen 22 entlang geführt, die eine Mehrzahl im Wesentlichen horizontal verlaufende Strömungsabschnitte des Strömungskanals begrenzen.

Hierbei kann die Temperierhohlwand 20 z.B. ein spiralförming um das Innere des Aufnahmebehälters 10 herum verlaufendes Strömungsführungselement aufweisen. Alternativ dazu kann, wie in den Figuren 6, 7 und 8 gezeigt, die Temperierhohlwand 20 mehrere Strömungsführungselemente 22 und/oder 21 aufweisen, die eine Mehrzahl einzelner, im Wesentlichen horizontal verlaufender übereinander angeordneter Strömungsabschnitte des Strömungskanals bereitstellen. In Übergangsbereichen zwischen diesen einzelnen im Wesentlichen horizontal verlaufenden Strömungsabschnitten des Strömungskanals fließt das Temperiermedium in eine im Wesentlichen vertikale Richtung (im gezeigten Beispiel im Wesentlichen vertikal nach oben) bis in den nächsthöheren, im Wesentlichen horizontal verlaufenden Strömungsabschnitt des Strömungskanals.

So fließt das Temperiermedium an den Strömungsführungselementen 22 entlang bis in eine am oberen Ende des Aufnahmebehälters 10 angeordnete Sammelkammer 23. Die Sammelkammer 23 weist einen Temperiermediumauslass 28 auf, aus dem das Temperiermedium aus der Temperierhohlwand 20 abgelassen werden kann. Der Temperiermediumauslass 28 kann im Wesentlichen rohrförmig ausgebildet sein. Ein oberes Ende des Temperiermediumauslasses 28 kann als ein Überlauf für das Temperiermedium ausgebildet sein und im Inneren der Sammelkammer 23 ein maximales Steigungsniveau des Temperiermediums definieren.

Oberhalb des oberen Endes des Temperiermediums kann sich in der Sammelkammer 23 Luft sammeln. Die Luft kann über einen Entlüftungsausgang 24 ausgelassen werden, der ebenfalls an der Sammelkammer 23 separat zum Temperiermediumauslass 28 angeordnet sein kann.

Durch diese Ausbildung wird verhindert oder zumindest reduziert, dass Luft über den Temperiermediumauslass 28 abgesaugt und zurück in den Temperierkreislauf geführt wird, was Fehlfunktionen und Störungen der Temperierung verursachen kann.

Die einzelnen Strömungsführungselemente 22 sind in der Seitenansicht als im Wesentlichen geradlinig ausgebildete Trennelemente angeordnet. Dabei sind die Strömungsführungselemente 22 im Wesentlichen horizontal ausgerichtet, weisen jedoch in der Seitenansicht in einem Winkel größer als 0° und bis zu 30°, bevorzugt von 1° bis 10°, besonderes bevorzugt von 3° bis 8°, nach oben von einem unteren Ende zu einem oberen Ende des jeweiligen Strömungsführungselements 22. Mit anderen Worten weisen die Strömungsführungselemente 22 (in der Seitenansicht und vom unteren Ende zum oberen Ende betrachtet) eine Steigung aus der Horizontalen heraus nach oben auf. Diese angeschrägte bzw. geneigte Ausbildung der Strömungsführungselemente 22 führt dazu, dass der Strömungskanal bzw. die Strömungsführung vom Temperiermediumeinlass 27 bis zum Temperiermediumauslass 28 durchgehend eine vertikal nach oben weisende Richtungskomponente aufweist. Der Strömungskanal verläuft somit insbesondere weder nach unten noch in eine ausschließlich horizontale Richtung, nicht einmal abschnittsweise. Deswegen bilden sich außerhalb der Sammelkammer 23 keine Lufteinschlüsse im Inneren der Temperierhohlwand 20. Gleichzeitig sind die Strömungsführungselemente 22 aber noch so flach angeordnet, also im Wesentlichen horizontal ausgerichtet, dass eine hinreichende horizontale Verteilung des Temperiermediums entlang der Innenwände der Temperierhohlwand 20 bewirkt wird.

**Figur 7** zeigt in einer Seitenansicht ein zweites Ausführungsbeispiel eines Aufnahmebehälters 10' zum Aufnehmen eines Einwegbeutels ohne Einblatttür 30 und ohne Außenwand. Dieser Aufnahmebehälter 10' weist in der Seitenansicht im Wesentlichen horizontal verlaufende Strömungsführungselemente 21 auf. Diese Strömungsführungselemente 21 ähneln - außer ihrer im Wesentlichen horizontalen Anordnung - den voranstehend beschriebenen Strömungsführungselementen 22.

Figur 7 zeigt somit eine alternative Ausbildung des Aufnahmebehälters mit im Wesentlichen horizontalen, nicht angeschrägten Strömungsführungselementen 21 (ohne Steigung).

**Figur 8** zeigt in einer perspektivischen, schematischen Darstellung den zweiten Aufnahmebehälter 10' ohne Einblatttür 30 und ohne Außenwand. Da sowohl das Bodensichtfenster 12 als auch die Türöffnung 36 für die Einblatttür 30 eine von unten bis oben spiralförmige Ausbildung der Strömungsführungselemente 21 stört, ist der Strömungskanal im Wesentlichen zweigeteilt. Der Strömungskanal weist die einzelnen im Wesentlichen in eine horizontale Richtung angeordneten Strömungsabschnitte zu beiden Seiten der Türöffnung 36 auf. Eine erste Anzahl der Strömungsführungselemente 21 definiert einen ersten, z.B. rechten, Strömungskanal vom Temperiermediumeinlass 27 bis zur Sammelkammer 23 und eine zweite Anzahl der Strömungsführungselemente 21 definiert einen zweiten, z.B. linken, Strömungskanal vom Temperiermediumeinlass 27 bis zur Sammelkammer 23, wo das Temperiermedium wieder aus der Temperierhohlwand 20 abfließt.

Allgemein können die Strömungsführungselemente 21 und/oder 22 einen oder mehrere Strömungskanale im Inneren der Temperierhohlwand 20 definieren und/oder begrenzen. Diese Strömungskanale können von einem einzigen Temperiermediumeinlass 27 zur selben, einzigen Sammelkammer 23 führen.

### Bezugszeichenliste

- 1: Vorrichtung
- 10: Aufnahmebehälter
- 10': Aufnahmebehälter
- 12: Bodensichtfenster
- 13: Kabelführung
- 14: Rührvorrichtung
- 15: Auffangwanne
- 16: Behälterwand
- 18: Rollen
- 19: Fixierfüße
- 20: Temperierhohlwand
- 21: Strömungsführungselement
- 22: Strömungsführungselement
- 23: Sammelkammer
- 24: Entlüftungsausgang
- 25: Isolierhohlwand
- 26: Temperierleitung
- 27: Temperiermediumeinlass
- 28: Temperiermediumauslass
- 30: Einblatttür
- 32: Türsichtfenster
- 33: Türverdunklung
- 34: Türscharnier
- 35: Türgriff
- 36: Türöffnung
- 40: Füllstandhöhe
- 41: Temperierhöhe
- 42: biologisches Medium
- 44: Einwegbeutel

## Patentansprüche

1. System mit einer Vorrichtung (1) zum Aufnehmen eines Einwegbeutels (44) mit:
- einem Aufnahmebehälter (10) mit einem Behälterinnenraum zum Aufnehmen des Einwegbeutels (44),
- einer Temperierhohlwand (20), die den Behälterinnenraum des Aufnahmebehälters (10) zumindest teilweise umgibt und
- einer Steuerung, mittels der der Druck eines in der Temperierhohlwand (20) angeordneten Temperiermediums auf maximal etwa 1 bar gesteuert und/oder eingestellt ist,
wobei das Temperiermedium zum Temperieren des Behälterinnenraums in der Temperierhohlwand (20) unter einem Druck von maximal etwa 1 bar aufgenommen ist.

2. System nach Anspruch 1 mit einer Temperiereinheit, die den Behälterinnenraum mittels des in der Temperierhohlwand (20) angeordneten Temperiermediums unter dem Druck von maximal etwa 1 bar temperiert.

3. System nach Anspruch 2, wobei die Temperiereinheit das Temperiermedium in der Temperierhohlwand (20) in einem geschlossenen Temperierkreislauf auf einen vorbestimmten Druck von maximal etwa 1 bar einstellt.

4. System nach einem der vorangegangenen Ansprüche, wobei das Temperiermedium zum Temperieren des Behälterinnenraums unter einem Druck von etwa 0,20 bar bis etwa 0,45 bar in der Temperierhohlwand (20) aufgenommen ist.

5. System nach einem der vorangegangenen Ansprüche, wobei in der Temperierhohlwand (20) zumindest ein Strömungsführungselement (21; 22) zur Strömungsführung des Temperiermediums durch die Temperierhohlwand (20) angeordnet ist.

6. System nach Anspruch 5, wobei das zumindest eine Strömungsführungselement (22) so schräg ausgebildet ist, dass die Strömungsführung des Temperiermediums durch die Temperierhohlwand (20) durchgehend mit einer vertikalen Richtungskomponente erfolgt.

7. System nach einem der vorangegangenen Ansprüche, wobei an einem oberen Ende der Temperierhohlwand (20) eine Sammelkammer (23) mit einem Entlüftungsausgang (24) und einem davon separaten Temperiermediumauslass (28) angeordnet ist.

8. System nach einem der vorangegangenen Ansprüche, wobei die Temperierhohlwand (20) so ausgebildet ist, dass sie zumindest ein unteres Drittel des Behälterinnenraums vollständig umgibt.

9. System nach einem der vorangegangenen Ansprüche, wobei die Temperierhohlwand (20) so ausgebildet ist, dass sie den Behälterinnenraum bis über eine vorbestimmte Füllstandhöhe (40) im Aufnahmebehälter (10) hinaus temperiert und/oder umgibt.

10. System nach einem der vorangegangenen Ansprüche, mit einem Entlastungsventil zum Einstellen und/oder Begrenzen des Drucks des Temperiermediums in der Temperierhohlwand (20).

11. System nach einem der vorangegangenen Ansprüche, mit einer elektrischen Heizung im Wärmeaustausch mit dem Temperiermedium.

12. System nach einem der vorangegangenen Ansprüche, wobei das Temperiermedium durch einen externen Wärmetauscher leitbar ist.

13. System nach einem der vorangegangenen Ansprüche, wobei an der dem Behälterinnenraum abgewandten Seite der Temperierhohlwand (20) eine Isolierung zumindest teilweise ausgebildet ist, die die Temperierhohlwand (20) nach außen isoliert.

14. System nach Anspruch 13, wobei die Isolierung zumindest teilweise in einer Isolierhohlwand (25) angeordnet ist, die an der dem Behälterinnenraum abgewandten Seite der Temperierhohlwand (20) ausgebildet ist.

15. System nach einem der vorangegangenen Ansprüche, wobei der Aufnahmebehälter (10) eine öffenbare Tür (30) aufweist, durch die der Einwegbeutel (44) in im Wesentlichen horizontaler Ebene in den Behälterinnenraum einbringbar ist.

16. System nach Anspruch 15, wobei der Aufnahmebehälter (10) im Wesentlichen in Form eines vertikal angeordneten Zylinders ausgebildet ist und die Tür (30) im Zylindermantel des Aufnahmebehälters (10) über einen Zylindersektor von etwa 80° bis etwa 150° ausgebildet ist.

17. Verfahren zum Aufnehmen eines Einwegbeutels mit den Schritten:
- Aufnehmen eines Einwegbeutels (44) in einen Behälterinnenraum eines Aufnahmebehälters (10) eines Systems gemäß einem der vorangegangenen Ansprüche,
- Temperieren des im Behälterinnenraum aufgenommenen Einwegbehälters (44) mittels eines in einer den Behälterinnenraum des Aufnahmebehälters (10) zumindest teilweise umgebenden Temperierhohlwand (20) angeordneten Temperiermediums,
wobei das Temperiermedium in der Temperierhohlwand (20) einen Druck von maximal etwa 1 bar aufweist.

## Claims

1. System having a device (1) for receiving a disposable bag (44), comprising:
- a receptacle (10) with a receptacle interior to receive the disposable bag (44),
- a hollow temperature control wall (20) that at least partially surrounds the receptacle interior of the receptacle (10) and
- a controller, by means of which the pressure of a temperature control medium arranged in the hollow temperature control wall (20) is controlled and/or adjusted to at most about 1 bar,
wherein the temperature control medium for controlling the temperature of the receptacle interior is accommodated in the hollow temperature control wall (20) at a pressure of at most about 1 bar.

2. System according to claim 1, comprising a temperature control unit that controls the temperature of the receptacle interior by means of the temperature control medium arranged in the hollow temperature control wall (20) at the pressure of at most about 1 bar.

3. System according to claim 2, wherein the temperature control unit adjusts the temperature control medium in a closed temperature control loop in the hollow temperature control wall (20) to a predetermined maximum pressure of approximately 1 bar.

4. System according to any one of the preceding claims, wherein the temperature control medium for controlling the temperature of the receptacle interior is accommodated in the hollow temperature control wall (20) at a pressure of about 0.20 bar to about 0.45 bar.

5. System according to any one of the preceding claims, wherein at least one flow guide element (21; 22) is arranged in the hollow temperature control wall (20) for guiding the flow of the temperature control medium through the hollow temperature control wall (20).

6. System according to claim 5, wherein the at least one flow guide element (22) is inclined so that the temperature control medium flows through the hollow temperature control wall (20) with a continuous, vertical direction component.

7. System according to any one of the preceding claims, wherein a collecting chamber (23) with a ventilation outlet (24) and a temperature control medium outlet (28) separate therefrom is arranged on an upper end of the hollow temperature control wall (20).

8. System according to any one of the preceding claims, wherein the hollow temperature control wall (20) is designed in such a manner that it completely surrounds at least a lower third of the receptacle interior.

9. System according to any one of the preceding claims, wherein the hollow temperature control wall (20) is designed in such a manner that it controls the temperature of, and/or surrounds, the receptacle interior to above a predetermined fill level (40) in the receptacle (10).

10. System according to any one of the preceding claims, comprising a relief valve for adjusting and/or limiting the pressure of the temperature control medium in the hollow temperature control wall (20).

11. System according to any one of the preceding claims, comprising an electric heater that exchanges heat with the temperature control medium.

12. System according to any one of the preceding claims, wherein the temperature control medium can be conveyed through an external heat exchanger.

13. System according to any one of the preceding claims, wherein an insulation is at least partially formed on the side of the hollow temperature control wall (20) facing away from the receptacle interior, the insulation insulating the hollow temperature control wall (20) from the outside.

14. System according to claim 13, wherein the insulation is at least partially arranged in a hollow insulation wall (25) formed on the side of the hollow temperature control wall (20) facing away from the receptacle interior.

15. System according to any one of the preceding claims, wherein the receptacle (10) has an openable door (30), through which the disposable bag (44) can be inserted into the receptacle interior in a substantially horizontal plane.

16. System according to claim 15, wherein the receptacle (10) is substantially designed in the form of a cylinder arranged vertically, and the door (30) is constructed in the cylinder shell of the receptacle (10) over a cylindrical sector of about 80° to about 150°.

17. Method for receiving a disposable bag, having the steps:
- receiving a disposable bag (44) in a receptacle interior of a receptacle (10) of a system according to any one of the preceding claims,
- controlling the temperature of the disposable pouch (44) received in the receptacle interior, by means of a temperature control medium arranged in a hollow temperature control wall (20) that at least partially surrounds the receptacle interior of the receptacle (10),
wherein the temperature control medium in the hollow temperature control wall (20) is at a pressure of at most about 1 bar.

## Revendications

1. Système avec un dispositif (1) pour recevoir un sachet à usage unique (44) comprenant :
- un contenant de réception (10) avec un espace intérieur de contenant pour recevoir le sachet à usage unique (44),
- une paroi creuse de mise en température (20), qui entoure au moins en partie l'espace intérieur de contenant du contenant de réception (10) et
- une commande, au moyen de laquelle la pression d'un milieu de mise en température disposé dans la paroi creuse de mise en température (20) est commandée et/ou réglée sur environ 1 bar maximum,
dans lequel le milieu de mise en température pour mettre en température l'espace intérieur de contenant est reçu dans la paroi creuse de mise en température (20) à une pression d'environ 1 bar maximum.

2. Système selon la revendication 1, comprenant une unité de mise en température qui met en température l'espace intérieur de contenant au moyen du milieu de mise en température disposé dans la paroi creuse de mise en température (20) à la pression d'environ 1 bar maximum.

3. Système selon la revendication 2, dans lequel l'unité de mise en température règle le milieu de mise en température dans la paroi creuse de mise en température (20) dans un circuit de mise en température fermé sur une pression prédéfinie d'environ 1 bar maximum.

4. Système selon l'une quelconque des revendications précédentes, dans lequel le milieu de mise en température pour mettre en température l'espace intérieur de contenant à une pression d'environ 0,20 bar jusqu'à environ 0,45 bar est reçu dans la paroi creuse de mise en température (20).

5. Système selon l'une quelconque des revendications précédentes, dans lequel au moins un élément de guidage d'écoulement (21 ; 22) pour le guidage de l'écoulement du milieu de mise en température à travers la paroi creuse de mise en température (20) est disposé dans la paroi creuse de mise en température (20).

6. Système selon la revendication 5, dans lequel le au moins un élément de guidage d'écoulement (22) est réalisé de manière inclinée de sorte que le guidage de l'écoulement du milieu de mise en température à travers la paroi creuse de mise en température (20) s'effectue de bout en bout avec une composante de direction verticale.

7. Système selon l'une quelconque des revendications précédentes, dans lequel une chambre de collecte (23) avec une sortie d'aération (24) et une sortie de milieu de mise en température (28) séparée de celle-ci est disposée sur une extrémité supérieure de la paroi creuse de mise en température (20).

8. Système selon l'une quelconque des revendications précédentes, dans lequel la paroi creuse de mise en température (20) est réalisée de sorte qu'elle entoure entièrement au moins un tiers inférieur de l'espace intérieur de contenant.

9. Système selon l'une quelconque des revendications précédentes, dans lequel la paroi creuse de mise en température (20) est réalisée de sorte qu'elle met en température et/ou entoure l'espace intérieur de contenant jusqu'au-delà d'un niveau de remplissage (40) prédéfini dans le contenant de réception (10).

10. Système selon l'une quelconque des revendications précédentes, comprenant une soupape de décharge pour régler et/ou limiter la pression du milieu de mise en température dans la paroi creuse de mise en température (20).

11. Système selon l'une quelconque des revendications précédentes, comprenant un chauffage électrique dans l'échange de chaleur avec le milieu de mise en température.

12. Système selon l'une quelconque des revendications précédentes, dans lequel le milieu de mise en température peut être guidé à travers un échangeur de chaleur externe.

13. Système selon l'une quelconque des revendications précédentes, dans lequel une isolation, qui isole la paroi creuse de mise en température (20) vers l'extérieur, est réalisée au moins en partie sur la face de la paroi creuse de mise en température (20) opposée à l'espace intérieur de contenant.

14. Système selon la revendication 13, dans lequel l'isolation, qui est réalisée sur la face de la paroi creuse de mise en température (20) opposée à l'espace intérieur de contenant, est disposée au moins en partie dans une paroi creuse isolante (25).

15. Système selon l'une quelconque des revendications précédentes, dans lequel le contenant de réception (10) présente une porte (30) pouvant être ouverte, à travers laquelle le sachet à usage unique (44) peut être introduit dans un plan sensiblement horizontal dans l'espace intérieur de contenant.

16. Système selon la revendication 15, dans lequel le contenant de réception (10) est réalisé sensiblement sous forme d'un cylindre disposé verticalement et la porte (30) est réalisée dans l'enveloppe cylindrique du contenant de réception (10) sur un secteur cylindrique d'environ 80° à environ 150°.

17. Procédé pour recevoir un sachet à usage unique comprenant les étapes :
- de réception d'un sachet à usage unique {44) dans un espace intérieur de contenant d'un contenant de réception (10) d'un système selon l'une quelconque des revendications précédentes,
- de mise en température du sachet à usage unique (44) reçu dans l'espace intérieur de contenant au moyen d'un milieu de mise en température disposé dans une paroi creuse de mise en température (20) entourant au moins en partie l'espace intérieur de contenant du contenant de réception (10),
dans lequel le milieu de mise en température dans la paroi creuse de mise en température (20) présente une pression d'environ 1 bar maximum.
